# EUROPEAN PATENT SPECIFICATION

(11) **EP 0 979 243 B1**
(45) Date of publication and mention of the grant of the patent: **02.08.2006**
(21) Application number: 98910872.5
(22) Date of filing: 18.03.1998
(51) Int. Cl.: C07K 7/08, C07K 14/435, G01N 33/68, A61K 38/10, A61K 38/17

(54) **ACTIVATION OF PEPTIDES**
AKTIVIERUNG VON PEPTIDEN
ACTIVATION DE PEPTIDES

(30) Priority: 18.03.1997 GB 9705519
(43) Date of publication of application: 16.02.2000
(73) Proprietor: Anmat Technology Limited, Delph, Oldham OL3 5DF (GB)
(72) Inventor: AJOULA, Harmesh, Singh, Saddleworth Oldham OL4 4AD (GB); CLARKE, David, John, Sandbach Cheshire CW11 1YB (GB)
(74) Representative: Jones, Graham Henry
(86) International application number: PCT/GB1998/000799
(87) International publication number: WO 1998/041535

(56) References cited:
- EP-A- 0 106 370
- WO-A-97/33908
- US-A- 4 874 710

## Description

This invention relates to the activation of peptides, polypeptides or proteins. For ease of reference in the following description the term peptide will be used, but it is to be understood that the invention is not limited to peptides since the invention finds equal utility in polypeptides, proteins and their derivatives and analogues and homologues thereof.

In particular this invention relates to peptides or derivatives thereof whose functions may be altered in a reversible and/or competitive manner by specific reactions. More particularly, the present invention relates to the modification of the functions of a peptide at an activated or naturally-active site of the peptide by the modification of an amino acid introduced at the said site, which amino acid is absent from the remaining peptide structure; and the introduction of another modifiable amino acid at a site known not to affect the activity or function of the peptide. The position of the sites may vary depending on the nature of the peptide being modified at such sites.

In particular, the method of this invention is particularly useful in relation to peptides capable of altering the integrity of lipid barriers and membranes, which may be activated by specific reactions.

Lipids are important in a range of biological, medical and industrial applications, particularly where a barrier needs to be maintained to aqueous materials. Living organisms also possess various lipid-bounded structures, typically involving phospholipid bilayer membranes, such as may be found in the cell membrane or plasmalemma of cells or their organelles such as mitochondria, lysosomes, nucleus, endoplasmic reticulum. The functions of the membrane include the retention of the cytoplasm inside the cell, maintenance of optimal conditions within the cell or its organelles, sensing the environment outside the cell and effecting appropriate bio-chemical responses within the cell or its organelles.

A number of agents are capable of breaching the barrier imposed by the lipid component of the cell membrane. Typically, these agents include detergents, which penetrate into the cell membrane disrupting its structure and eventually dissolving the lipid molecules. Some enzymes, typically phospholipases, may also disrupt lipid membranes. Other proteins, polypeptides and peptides may also disrupt or breach the barrier, or are involved in the permeation of water soluble materials across lipid membranes. Natural biological membranes of the cell possess various proteins whose prime function is either to form an aqueous channel (or pore) or to act as a carrier to transport molecules from one side of the membrane to the other.

The central importance of lipid membranes to the integrity and function of biological cells has been exploited in various natural processes which seek to damage or destroy such cells. A range of toxins have been identified, which disrupt cell membranes. Typically, these toxins are called cytolytic toxins and many are based on peptide structures. Some of these cytolytic toxins act first on the protein components in the cell membrane of biological cells. Typically, these are larger proteins or polypeptides such as cholera toxin or botulinum toxin.

WO97/33908 describes peptides with lytic activity having at least an amphipathic α-helix of sufficient length and character to function lytically.

EP0106370 relates to an assay for an analyte using a ligand coupled to a liposome lysing agent.

US4874710 relates to a peptide with lytic activity having a N- or C-terminal comprising one or more moieties which result in increased positive charge.

The method of this invention is particularly useful when it is desirable to use lower molecular weight polypeptides and peptides acting on the lipid component of membranes. Such polypeptides and peptides may act by forming channels in the membrane or by physically disrupting the membrane, which processes may involve co-operative behaviour between the polypeptide or peptide molecules forming aggregates in the membranes. Preferred examples of this type of toxin are the lysins produced by pathogenic bacteria, a group of peptides called defensins, produced by a range of insects and mucosal cells in mammals, and also by cells involved in defence against pathogens, such as neutrophils and macrophages. The most studied of this group of peptides and the most preferred peptide for use in this invention is melittin, which is the main toxin in bee venom, together with phospholipases. Melittin functions by permeabilising membranes.

Whilst there have been many basic scientific studies on the defensins, comparatively few applications have developed. Defensin peptides have a broad specificity, and are being developed as broad spectrum anti-microbials for topical applications such as contact lens fluids. Defensins also seem to promote wound healing. Pro-defensins and defensins from human and other sources are being cloned. Some are being considered for expression in plants to protect against microbial infection and diseases in plants.

(Litchfield et al Clin. Chem. (1984) 30: 1441-1445, EP-A-0106370, developed an immunoassay based on the cytolytic action of these toxins on liposomes. In that immuno assayhaptens were chemically coupled to a cytolytic peptide (cytolysin) such that on incubation with antibodies (binder) the activity of the peptide decreased to low or insignificant levels. When this reaction was carried out in the presence of analyte similar to the hapten, the binding of the antibody to the cytolytic peptide was competitively inhibited making the peptide active.. The action of the uninhibited cytolytic peptide on liposomes caused release of various detectable labels such as fluorophores, enzymes etc. from the lipsosomes resulting in the development of a measurable signal. However, this assay procedure could not be developed for a wide range of analytes primarily because conjugation of the haptens to the cytolysin either resulted in its inactivation or a limited inhibition of its cytolytic activity on incubation with antibodies or other similar binding proteins. These problems particularly result from the presence of a number of potential amino acid sites in the peptide structure suitable for chemical modification and coupling of haptens and other agents. This illustrates the difficulty of using natural peptides with multiple modifiable sites for developing such applications.

Litchfield et al's assay although shown to be useful for few analytes has serious limitations. One limitation is that the coupling of haptens to naturally occurring cytolysins does not always produce conjugates having adequate activity. For example, permethylation of melittin results in almost complete loss of activity (Biochem. J. (1992) 284, 663-665). Another limitation is that the active conjugate can not always be effectively inhibited by the binder thus giving a high background signal. Background signal is the signal obtained for the inactivated form of the peptide. Further, the sensitivity of assay is limited because more than one hapten is usually present on the conjugate due to natural cytolysins often bearing more than one chemically modifiable functional group. Yet another limitation is significantly lowered aqueous solubility of the conjugates especially when hydrophobic haptens are used.

It is an object of this invention to solve the problems of known immunoassay techniques and consequently to extend the scope for assays. The invention is also applicable to other areas such as therapeutics.

According to one aspect of the invention there is provided a method of modifying the activity of a peptide, the method comprising:
a. taking a peptide comprising an active site and a non-active site, the non-active site being capable of conjugation with another particle or surface; and
b. substituting a first amino acid at a first site in the active site of the peptide with an amino acid which is modifiable by a reaction whereby the active site of the peptide is inactivated; and
c. substituting other chemically modifiable amino acids in the peptide with amino acids which are not modifiable by the reaction;
wherein the peptide is selected from or derived from lytic peptides, melittin, gramacidin, alamethicin, paradixin, ceropin, maganins, C18G, P14A, the peptide sequence:
LLQSLLSLLQSLLSLLQWLKRKRQQ, peptide sequence: GFFALIPKIISSPLFK, delta-Hemolysin, Mastopparan, Bombolitin, Crabolin, KLLKLLLKLLKLLLKLLLKLLKKLLKLLLKLLLKLLK, Defensins, antibacterial lytic peptides, Paradaxin, Cationic peptides, amphipathic peptides, GALA, KALA, HELP, LAGA, influenza Hemagglutinin peptide, HIV fusion peptide, peptide sequence: FEAALAEALAEALA, Signal sequences; and P-25
and wherein when the peptide is exposed to the reaction, the active site is reactivated by modification at the first site.

The activity of the peptide may be cytolytic activity or membrane activity. The reactivation of the active site may be delayed reactivation, the reactivation preferably being delayed for at least 14 hours. The particle or surface may be a lipid membrane and the peptide may be integrated with the lipid membrane.

Preferably, the non-active site capable of conjugation is introduced into the peptide by substitution of at least one amino acid at a non-active site of the peptide. All the other amino acids may be replaced in the peptide. The peptide may be obtained or derived from natural peptides or polypeptides.

Preferably, the peptide has been produced synthetically.

Preferably, the active site of the peptide is modified using an ionisable group, a redox sensitive group, an affinity ligand, an enzyme substrate, a light sensitive group, a pH sensitive group or a chemical group sensitive to physical, chemical or biochemical effects.

The peptide is preferably modified by the replacement of amino acids with other amino acids or groups whereby binding of the peptide to a lipid membrane is improved.

Typically, this invention concerns the modification of biologically active peptides, such as hormones, drugs, toxins, and peptides which act on lipid bilayer membranes. The active site as defined by this invention need not be the same as the active or functional site as will be understood by those expert in the field, that is it may be a naturally occurring active site or an artificially introduced activated site. The active site of a peptide may be determined by a number of well known methods, and is typically determined by synthesising a range of peptides each housing a modifiable amino acid placed at a different position and determining the alteration of the activity or function of the peptide or its conjugate with ligands, upon binding to other molecules such as antibodies, upon cleavage of the ligand, or upon effecting changes physically, chemically, enzymically or by other means at the site. The position of an active site may depend on the nature of molecules attached at the site. By the same means, a modifiable amino acid may be placed at a site so determined so as not to affect the function or activity of the peptide. This modified amino acid may possess different chemical properties to the first amino acid which it replaces.

Preferably the invention uses synthetic peptides where chemically modifiable amino acids (for example lysine, cysteine, serine, threonine, tyrosine, aspartate, glutamate etc.) or amino acid analogues (such as ornithine) have been eliminated. Such modifiable amino acids may be eliminated from the structure completely or may be replaced by non-modifiable amino acids such that the activity of the peptide is not significantly altered or may, in fact, be increased. Easily modifiable amino acids (for example lysine) may then be introduced into the peptide structure where desired. An important aspect of this invention is the introduction of such modifiable amino acids at either position altering the function or activity of the peptide or at a site in the peptide structure which does not affect the activity or function of the peptide or a combination of both aforesaid sites. Thus, if the peptide has a modifiable amino acid at a site affecting its membrane active function, its modification or consequences thereof results in the efficient inactivation or inhibition of the activity of the peptide by binding to a binder. Preferably, this involves the introduction of a modifiable amino acid at the site and its chemical modification with a ligand, such that binding of an antibody or another binding protein to the peptide efficiently inhibits the activity of the peptide.

The method of the invention may also involve chemical modification of a peptide which results in a change of activity in the peptide, for example the reversible inactivation of the peptide. For example, the modification may be effected using an ionisable group or groups whose reversible reactions with protons make the peptide responsive to pH, or, similarly, by modification with a radox sensitive group making the peptide sensitive to its redox environment. One example of labile bonds would be citraconylation at the active site and then using acidity of media to activate the peptide. Another example is the modification of the activated site to include an enzyme substrate, such that on contact with the enzyme, and on subsequent enzymatic reaction, the peptide is activated. For example by coupling a bulky group (for instance another short peptide or sugar or nucleic acids, DNA, etc.) at active site to inactivate the peptide, cleavage of the bulky group by specific enzyme will then activate the peptide. A further example is the introduction of chemical group sensitive to physical effects. For example a light sensitive group whose structure may be changed by photochemical reaction or by photoisomerism or light stimulation can be used. An example of light sensitive group would be to incorporate lightswitchable moiety such as azabenzene or light switchable amino acids (Bioorganic & Medicinal chemistry letters (1994), 4, 2145-2146) in order to regulate the peptide activity by structural changes. By these means, the activity of the peptide can be modified by specific physical, chemical, biochemical and biological reactions.

Another aspect of this invention is the modification of a peptide at a site which does not affect its activity or function. In this way a peptide can be readily coupled with other molecules, materials or to surfaces. Furthermore, other well-known reactions can be used to target the peptide to specific sites. For example, a peptide can be coupled to specific chemical functional groups in the material, or to specific sites on its surface and, by the coupling of other structures to the peptide, it can specifically bind to other biological structures, for example to a specific receptor on the cell surface.

Accordingly, the invention further provides peptides whose activity is controllable by specific reactions and whose activity can be targeted to specific sites. Such peptides may be highly useful in diagnostics, detection, sensors, therapeutic or other pharmaceutical applications. Such peptides offer a considerable improvement over the prior art. It is possible using the peptides of the invention to develop immunoassays for a wide range of potential analytes by very straightforward means. The presence of only one activated site in a peptide, results in higher sensitivity and it also results in a higher efficiency of interaction of the peptide conjugate by the aforesaid physical, chemical, biochemical or biological reactions resulting in a much lower backgroup activity compared to modification of peptides at other similarly modifiable sites that may exist. In the case of antibody binding to the activated site, when using the peptides of the present invention, it is possible to use only one molecule of antibody to bind to one molecule of peptide to achieve efficient, inhibition of peptide activity with low residual or background activity. Thus, on the introduction of labels such as fluorophores and enzymes into membrane bounded particles such as liposomes, a wide range of test and assays can be developed. The examples which follow illustrate typical uses of such peptides in such assays.

Preferably, as the active site can be chemically located at almost anywhere along the sequence of the peptide, the best position may be selected by producing a series of analogues for a given analyte, or the most effective analogue can be developed further to form the basis of an assay for that particular analyte or for the other aforesaid applications.

Another characteristic of the use of peptides of this invention is the ability to introduce positive feedback amplification into the process of activation of the peptide. Typically, the activity of the peptide is blocked such that on release of activator from membrane bounded particles, higher activities of peptides are produced, which in turn release more activator resulting in a positive feedback effect and non-linear or exponential release of bio-active material or diagnostic marker. For example, the peptide can be modified by binding to it a biotin analogue such that binding of streptavidin or avidin results in the blocking of activity until such times as biotin or a similar analogue is released which competes with the streptavidin or avidin activating the peptide. Similarly, the peptide may be inactivated by incorporation of a moiety at the activate site such that on release of an enzyme from the particles the peptide is activated. (For example, proteases, and glucuronidases may be suitable enzymes), to activate suitably modified peptides. Alternatively, modified binding proteins may be used which are relatively easy to release from the peptide. For example a biotin-modified peptide can be blocked by binding to nitroavidin until such time as the presence of free biotin activates the peptide by binding with the nitroavidin. Similarly changes in pH can affect the binding of proteins (For example nitroavidin) to a modified peptide (For the above example biotin-peptide conjugate) and this can be useful parameter to effect activation/inactivation of the peptide. It is further possible to include more than one activation means on the peptide by inducing activity using both a specific ligand binding reaction and another parameter for instance pH change. In this respect, for instance a pH modified peptide can be specifically activated by ligand binding reaction at the activated site and only when both conditions are met the peptide becomes active. Peptides can be modified such that activity can be controlled by altering other parameters for instance redox change, photochemical means, enzymatic reactions, acid or alkali labile bonds, chemically cleavable bonds.

In these respects, another characteristic of this invention is the modification of the peptide such that it remains associated with the lipid membrane. Typically, this involves the introduction of hydrophobic amino acids or modification with a hydrophobic or amphiphilic chemical species, such as fatty acids (for example myristic acid). Following such modifications, the peptide remains tightly attached or integrated into the lipid membrane, whilst retaining its ability to be specifically activated by the aforementioned reactions. Integration of the peptide into the lipid membrane is particularly useful when used in diagnostic assays and tests because the reagents need not be added separately providing one reagent (i.e. a liposome containing marker and peptide bound to the membrane) for the test. In the case of diagnostic applications involving complex biological samples and therapeutic applications, it is important that such peptides are not left free to interact with other membranes in the sample or body which would prevent their specific action on the lipid membrane bound diagnostic drug delivery systems.

There are a number of chemical procedures well known in literature (For example, Bioconjugate Techniques (1996), Greg, T, Hermanson Academic Press, USA and references therein) which can be used to conjugate molecules together by chemical bonds. The particular attachment chemistry used will depend on the nature and reactivity of functional groups present on both the ligand and the peptide to be conjugated. For instance activated esters such as n-hydroxysuccinimide ester, snf p-nitrophenyl ester can be used to couple carboxyl groups with primary amino groups. Alternatively, homobifunctional cross-linkers (for example glutaraldehyde can be used to conjugate two amino groups) or heterobifunctional cross-linkers (for example m-maleimidobenzyl-n-succiminide may be used to conjugate thiol and amino groups) may be used. Different spacer arms can be incorporated between the two conjugating molecules either by using cross linkers or separately. Activating agents such as carbodiimides, or BOP could be used to link molecules together. Bonding methods such as oxidation of thiols to disulfides can also be used. Additionally it will be apparent to those skilled in the art that bonding may be carried out by non-covalent interactions such as salt bridges or hydrophobic interactions.

Whilst specific reactions involving changes in pH or redox can result in the reversible activation or inactivation of the peptide, and chemical or enzyme catalysed biochemical reactions or photochemical means can result in the irreversible activation or inactivation of the peptide, the reactions of binding proteins such as antibodies normally require a competitive reaction. In the latter case, the ligand or chemical species competing with the hapten to bind to the protein must be present at the same time resulting in a reversible activation or inactivation of the peptides. It is a further aspect of this invention, that the binding of the peptide to the ligand can be performed in advance of its use such that an inactivated peptide is produced. This results in a stable material where all the significant components are integrated into the same complex, such that the peptide may be subsequently activated by displacement of the binding protein. Comparable heterogeneous immunoassays and competitive immunoassays require the separate addition of each reagent, resulting in relatively complex multi-step procedures. In particular, this has prevented therapeutic applications of such reactions, where it is often impractical to add the reagents separately and at different times.

By the above means, this invention provides for a homogeneous peptide activation system which may be incorporated into a particle for the purposes of diagnostic assay and sensing, or the controlled release of drugs from particulate delivery systems where the inactivated peptide may be used directly or within a particle. In the case of such an integrated system, it may be particularly important to achieve a low residual or background activity of the peptides, otherwise diagnostic signal or bio-active agents would be steadily released from the particle.

The invention also allows the incorporation of the more than one function into peptides. For instance a peptide that is active at low pH, for instance GALA (J.Biol.Chem. (1988), 263, 4724-4730), HELP (Protein Eng. (1992), 5, 321-31) may be conjugated to a ligand. At high pH the peptide conjugate will then be activated by pH and also ligand binding. Thus, more than one specifically activated function may be introduced into the peptide. When specific reaction with both such functionalities is require to activate the peptide, much lower levels of residual or background activity can be achieved. Typically, the background activity of an inactivated peptide achieved by this invention is of the order of a few percent. If the background activity of the second functionality is also of a similar order, the background is the result of both. It is, of course, quite possible to introduce further functionalities into the peptide structure, such as the aforementioned modifications at sites not affecting the function of the peptide.

Embodiments of the invention will now be described in more detail, by way of example only, with reference to the following examples and the accompanying drawings Figures 1 to 7 in which:
Figure 1 is a plot of fluorescence against time and shows the results typical inhibition by antibody binding at the hapten site of peptides in Example 1;
Figure 2 is a plot of fluorescence against time and shows the quantitative detection of biotin and PETN;
Figure 3 is a plot of fluorescence against time and shows the displacement of binder;
Figure 4 is a plot of fluorescence against time stability of cloaked peptide and its uncloaking by addition of ligand;
Figure 5 is a plot of fluorescence against time and shows activation of peptide by redox switching;
Figure 6 illustrates the release of a marker from a liposome under the control of a modified peptide in accordance with the invention; and
Figure 7 illustrates a modified peptide in accordance with the invention.

### Example 1: Preparation of peptides

a) A short peptide of sequence:
   LLK LLL KLL LLK LLK-amide
   was prepared by solid-phase synthesis using Boc chemistry developed by Merrifield. 4-Methylbenzhydrylamine resin (0.5mMoles) was used as the starting material. The side chain protecting groups which were used were: Arg (Tos) and Lys(2-CIZ). Each synthetic cycle consisted of (i) a 2min and 25min deprotection with 50% Trifluoroacetic acid in Dichloromethane (ii) neutralisation with 5% Diisopropylethylamine/Dichloromethane and (iii) coupling with 1.5mMoles amino acid, 1.5mMoles BOP Castro's reagent) and 4.5mMoles N.N diisopropylethylamine (DIPEA) in dimethylforamide (DMF) for 40 mins. A second coupling was used when necessary to drive the reaction to almost completion (>99.8%).
   Myristic acid was coupled to the peptide in an analogous manner to amino acids. At the end of synthesis the peptide was cleaved with HF by the procedure of Stewart J. M. and Young, J. D. (1984) Solid-Phase peptide synthesis 2nd Ed. Pierce Chemical Co. Rockford IL. The peptide resin was then treated with 20ml HF, 0.5g thiocresol and 0.75g p-cresol and, after evaporation of HF, extraction was carried out with 50% acetic acid/water. The peptide was purified on C-8 reverse phase Vydac semi-prep column using linear gradient of 20% acetonitrile/0.1 % TFA to 80% acetonitrile/0.1% TFA over 45 mins. The product peak was lyophilised.
   The peptide was then modified by replacing all Lys residues with Arg and blocking the C and N-terminal residues and then coupling the fatty acid myristic acid to its N-terminus. The fatty acid also helps in the binding of peptide to liposomes and attachment at the N-terminus away from active site does not cause serious problems in retaining adequate activity. A Lysyl was introduced at a critical site to produce the modified version:
   Myristoyl-LLR LLL RKL LLR LLR-amide.
b) A longer natural peptide (melittin) having the sequence
   GIG AVL KVL TTG LPA LIS WIK RKR QQ-amide
   was modified to
   Acetyl- GIG AVL RVL TTG KPA LIS WIR RRR QQ-amide
   by replacing all natural Lys residues with Arg, blocking the N-terminal with acetyl and introducing new Lys residues at critical sites on the peptide. The modified analogue was prepared in a similar manner to the peptide produced in Example a) except that the N terminal was reacted with acetic anhydride (6 fold excess) to acetylate the terminal amino group.

Other peptides such as the ones below can be produced by identical procedures to those given above.
Myristoyl- GIG AVL RVL TTG KPA LIS WIR RRRQQ-amide
Myristoyl- GIG AVL RVL TTG LPK LIS WIR RRRQQ-amide
Myristoyl- GIG AVL RVL TTG LKA LIS WIR RRRQQ-amide
Myristoyl- GIG AVL RVL TTG KPA LIS WIR RQQ-amide
Myristoyl- LLQ SLL SLL QSL KSL LLQ WLR RRR Q-amide

### Example 2: Preparation of liposomes encapsulating marker

As an example of particulates for use in drug-delivery or diagnostic assay,liposomes encapsulating Calcein (Sigma) dye were prepared by the extrusion method of Biochim. Biophys. Acta (1985) 812; 55. Specifically, Phosphotidylcholine (12mg) and cholesterol (2mg) were evaporated to form a lipid film in a round bottom flask. The film was hydrated with 2ml of 120mM Calcein in 10mM sodium phosphate/120mM Sodium chloride buffer of pH 7.4. Liposomes were then prepared by ten times extrusion through a 0.2 micron polycarbonate filter using the reported method. Any non-encapsulated dye was removed by gel filtration on a PD-10 column.

Liposomes containing other markers including enzymes (for instance alkaline phosphatase, Glucose Oxidase, HRP, Glactosidase, asparginase) can also be prepared in a similar manner and used. Where liposomes containing enzymes are used in diagnostic assays, the signal is usually provided by its action on substrates.

### Example 3: Modification of peptides through attachment of molecules to the peptide

a) In the example below a preactivated ester is used to couple biotin at only one position only.
   A peptide (0.02mmole) prepared as described in Example 1, was dissolved in 4ml DMF and Biotin N-hydroxysuccinimide (0.1mmole) is added followed by DIPEA (0.3mmole). The reaction is allowed to proceed until completion as judged by a Ninhydrin assay (Stewart J. M and Young, J.D. (1984) Solid-Phase peptide synthesis, 2nd Ed. Pierce Chemical Co. Rockford IL). The DMF is then removed by vacuum and the conjugate of the peptide and biotin was purified by reverse phase HPLC (see above).
b) Ferrocene labelled peptide was synthesised by conjugation of ferrocenecarboxylic acid with peptide in example 1b using BOP activation in solution phase. Peptide (10 umoles) was dissolved in 2ml of DMF and added to 5ml solution of ferrocenecarboxylic acid (100umoles) and BOP (100umoles) under stirring. N,N-diisopropylethylamine (0.3mmole) was added next and reaction allowed to take place for 2 hours at room temperature. The solvent was removed by vacuum and product purified by reverse phase HPLC using C-8 column. The mono labelled product was isolated and characterised by electrospray mass spectroscopy. The resulting ferrocenic peptide could be chemically reduced by simple addition of sodium dithionite.
2 -Imminobiotin was conjugated to a peptide prepared as in example 1a using its N-hydroxysuccinimide ester in identical manner that is described above in relation to to biotin.
d) A PETN analogue can also be conjugated as N-hydroxysuccimide ester using similar method.
e) In another variation the peptides in examples 1a could be synthesised with the amino acid Cysteine instead of lysine. With such peptides ligand could be conjugated using maleimide activation. For instance Biotin-maleimide (Sigma) could be used to couple the biotin.
f) Similarly peptides can be synthesised with carboxylic groups (aspartic acid, glutamic acid) which can be activated (using reagents such as carbodiimides, BOP) to react with ligands.

### Example 4: Effective inhibition of conjugate activity with binder

In a typical assay a 2ml buffer containing a known concentration (depending on the activity) of a peptide prepared as in Example 1 is treated with 3µl liposomes prepared as in Example 2. The progress of dye leakage from the liposomes indicates release of the marker and is followed by recording an increase in fluorescence signal. The assay can be repeated in the presence of a binder. Where Calcein is used as the dye excitation and emission wavelengths of 490nm and 520nm were used. The assay can be repeated in the presence of binder. The concentration of binder required to fully bind the peptide can be evaluated using series of binder concentration at fixed peptide concentrations or vice versa.

In a typical case, a peptide solution is prepared at 0.1 to 0.01mg/ml (several fold lower concentrations are used for a higher activity peptide) and a 10µl volume of the peptide solution is added to 2ml buffer such as 20mM Tris-HCL containing 120mM NaCl pH 7.5. At this stage aliquots of binder protein at stochiometric or above levels can be added and incubation allowed to take place. A response is obtained by adding 3µl aliquots of liposomes typically prepared as in Example 2 while the fluorescence is recorded continuously. In some experiments, it is desirable to add liposomes first and then initiate signal by addition of peptide, conjugate or complex with binder. There can be several variation to these steps. For instance in the case of displacement experiments to show activation of the peptide, the peptide conjugate and binder are mixed in a 2ml assay volume and then liposomes are added. The activation is then achieved by addition of an aliquot of the activating ligand at desired time.

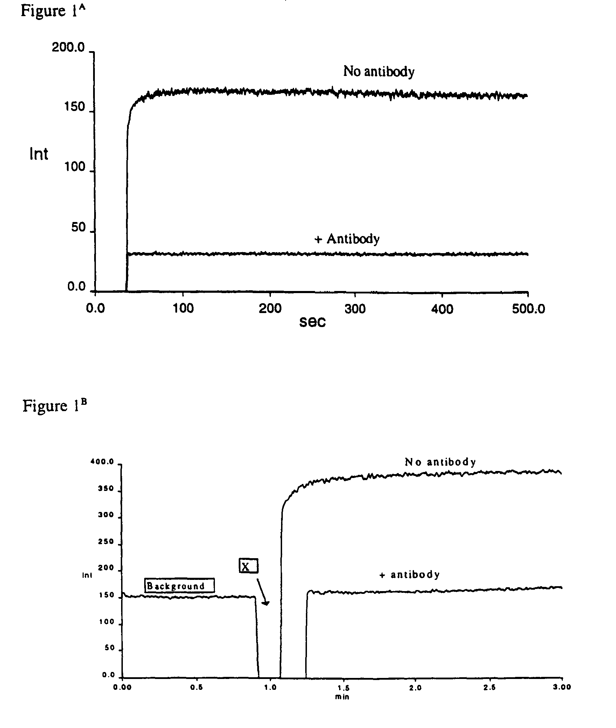

In the experiment represented by figure 1A antibody is used as binder and PETN hapten attached to the peptide from Example 1b is used as the conjugate. In theexperiment represented by Figure 1B an anti-biotin antibody is used as the binder and biotin attached to peptide prepared in example 1a is used as the conjugate.

The results of the assay are shown in Figure 1. As Figure 1 shows, the binding is so effective at this site that the background signal caused by the peptide conjugate is almost indistinguishable from natural background from liposomes alone (within the lower trace).

### Example 5: Modulation of activity by presence of ligand

A 2ml volume of buffer containing a known concentration of ligand is added followed by addition of binder, (typically the binder to peptide conjugate ratio is arranged to be slightly in excess over 1:1 stochiometry) and peptide conjugate. Liposomes (3µl) prepared as described in Example 2 are then added and the progress of lysis of the liposomes is recorded as before. The signal obtained was found to be proportional to the analyte concentration.

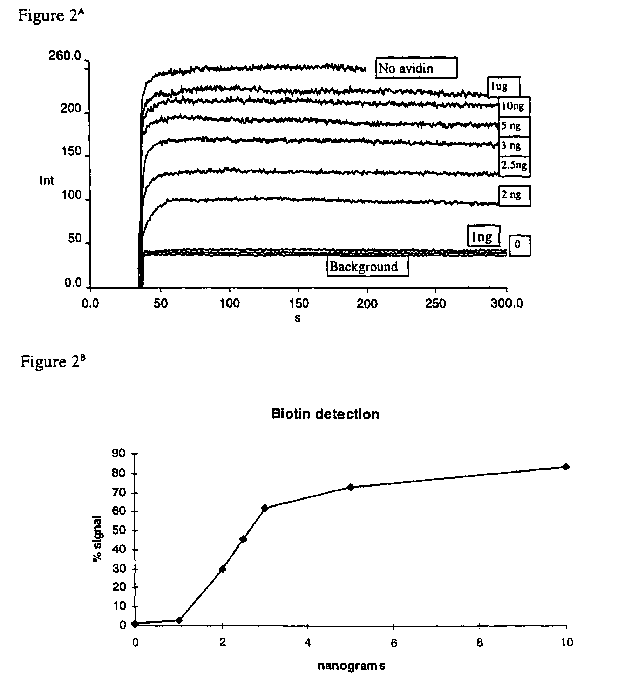

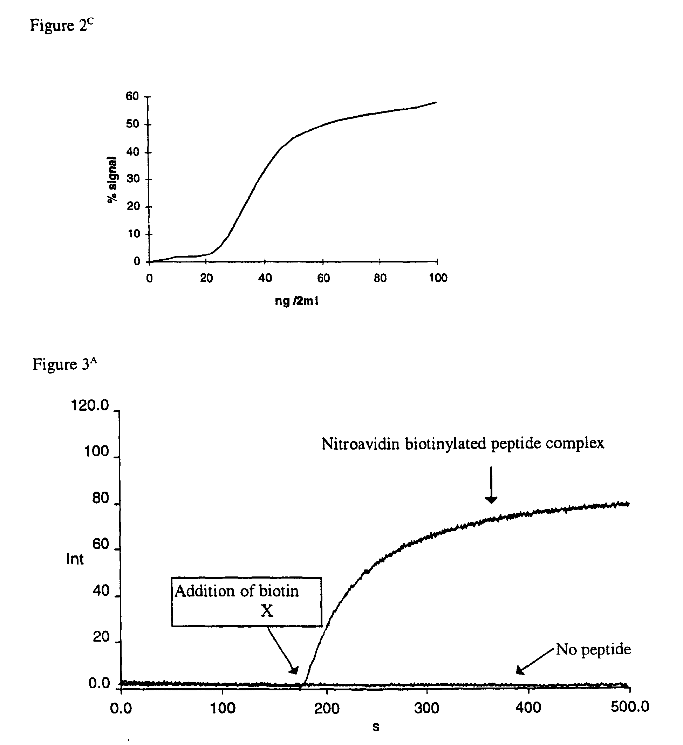

In the experiment represented by figure 2A the binder avidin containing the indicated levels of biotin ligand is used to bind to peptide of Example 1b after modification with biotin. Figure 2B is a plot of response verses biotin concentration for the same system. Similarly Figure 2C shows the quantitative detection of PETN using the peptide from Example 1b after conjugation with PETN analogue and using anti-PETN antibody as the binder.

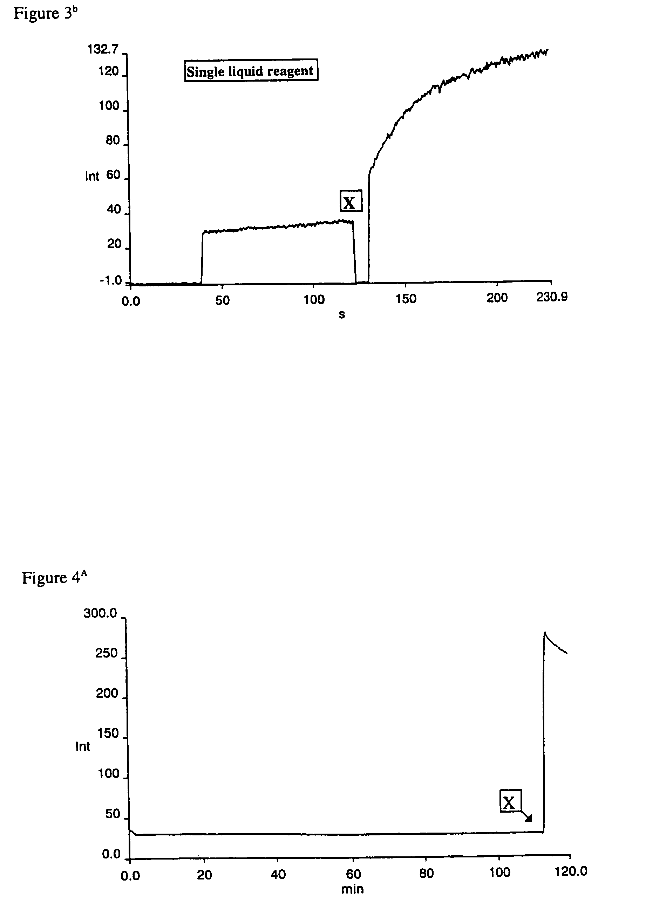

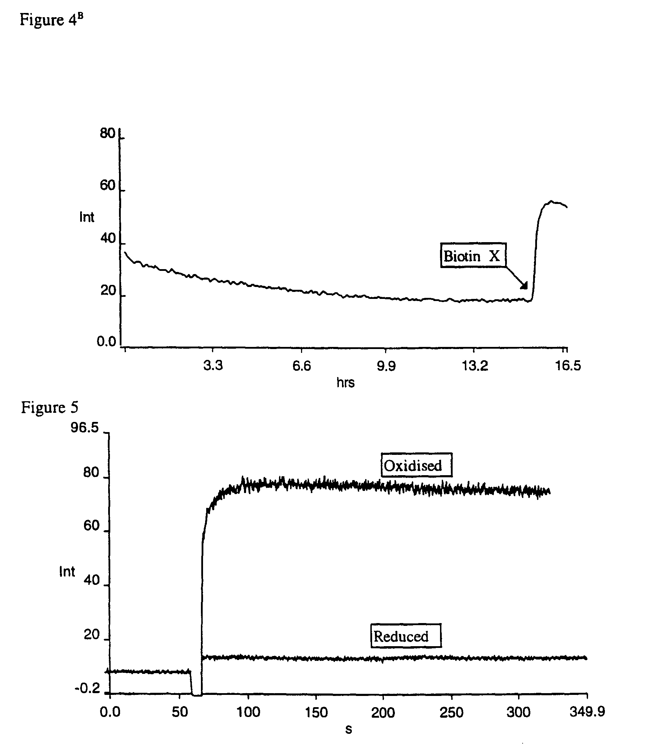

To assess the long term stability of blocked biotinylated peptide 2ml buffer containing nitroavidin biotinylated peptide complex and 3µl of Calcein-containing liposomes prepared as in Example 2 were incubated overnight and Biotin added next day. Dye leakage followed continually throughout. The results are shown in Figure 4B.

### Example 6: Regeneration of activity by displacement of the binder

In a typical use the peptide conjugate is bound to the binder and liposomes (3ml) added as above. The progress of lysis of the liposomes is recorded and then the binder is displaced by addition of free ligand of a similar structure to the ligand conjugated on the peptide thereby resulting in the release of free active peptide conjugate.

For assays in which biotin was used to activate the peptide the Nitroavidin- biotin peptide complex was pre formed in 2ml of 10mM sodium phosphate+140mM NaCL buffer pH 7. Calcein-containing liposomes (3µl) were then added to the buffer. After achieving the blocking of peptide (as indicated by a stable baseline on a plot of fluorescence against time) biotin was then added to reactivate the peptide by displacement. A control experiment was conducted in the absence of peptide.

In an experiment represented by figure 3A, a biotin conjugate prepared using the peptide in example 1b was bound with nitro-avidin and unbound by addition of biotin as the displacing ligand.

As shown in Figure 3 when biotin is added at point X, it causes rapid activation of peptide thereby resulting in release of fluorescent detectable label from the liposomes.

Figure 4A shows the stability of integrated system (single reagent) in the inactive form for under 2hrs. All the reagents namely the biotin modified peptide of example 1b, avidin, and liposomes containing calceium dye are added in 2ml buffer and signal recorded continually. Full lysis of the liposomes was obtained by adding triton-X100 at point X. The figure shows the system can be made very stable with little or no leakage of the contents.

Figure 4B shows the stability and activation of integrated system form for under 17hrs. All the reagents namely the biotin modified peptide of example 1b, nitroavidin, and liposomes containing calceium dye are added in 2ml buffer and signal recorded continually. Activation of the peptide is achieved by adding biotin at point X. The figure shows the system can be made stable and activated specifically with ligand.

**Example 7: Preparation of nitroavidin** Nitroavidin was prepared by a published method (Biochem .J. (1996) 316,193-199). 10mg of avidin in 2ml of 50mM Tris-HCl buffer at pH 8 was treated with 20mM of tetranitromethane for half an hour at room temperature. The sample was dialysed against 3L of 1M sodium chloride and twice against water. The biotin binding sites of the nitroavidin were then blocked with 0.6mM biotin solution at pH 4 using 50mM citrate buffer. The solution was then brought to pH 10 to release biotin from the modified sites. Extensive dialysis against 20mM sodium carbonate pH 10 buffer followed by dialysis against distilled water yielded nitroavidin with reversible biotin binding activity.

### Example 8: Redox activation of peptide

In a typical experiment a ferrocenic form of peptide in example 1b prepared as in Example 3b was dissolved at a concentration of 0.01mg/ml in water. A 10µl aliquot of the peptide solution was added to 2ml assay buffer with the presence and absence of reducing agent (sodium dithionite). A 3µl aliquot of liposomes was used for these assays. Figure 5 shows the activation of redox peptide by switching the oxidation state from low (lower trace) to high (upper trace).

### Example 9: Diagnostic and therapeutic applications of controlled release of marker

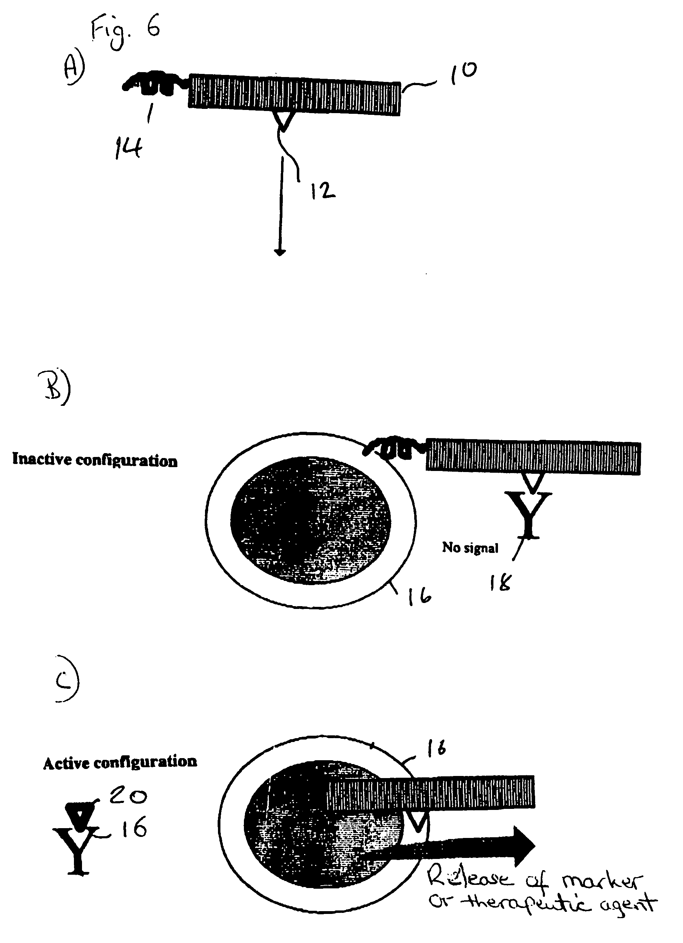

Fig. 6 shows a peptide 10 which has a first site 12 which confers a particular functionality on the peptide, e.g cytolysis. That site 12 is "cloaked" by binding of a binder 18, such as an antibody, to the site or to a modified amino acid adjacent the site or another binder such as nitroavidin where the peptides has been biotinylated as described in Example 3. the cloaked site 12 is inactive. The peptide 10 also comprises a second site 14 which facilitates binding of the peptide to membranes. The second site 14 may be naturally occurring in the peptide or may be introduced for example by introducing hydropobic residues at the respective end of the peptide.

Also provided are liposomes 16 containing a detectable marker, in the case of diagnostic applications or a pharmacuetical in the case of therapuetic applictions.

In the inactive configuration shown in Fig. 6B, binder 18 binds to the peptide at the first site 12 inhibiting the function of that site. The peptide 10 can also bind to the liposome 16 through its second site 14.

In the presence of an activator 20 (which in the case of diagnostic applictions might be an antigen which is the analyte of interest or in the case of therapeutic applications might be an antigen carried by cell type which is to be treated) compeptition occurs between the binder and the first site and the activator (as the peptide has been modified to include a binding site which mimics the activator) such that the binder 18 binds preferentially to the activator 20. The active site 12 of the peptide is thus exposed and the membrane lysis function of the peptide causes it to disrupt the membrane of the liposome 16. The marker or therapeutic agent is released from the liposome.

Accordingly, the invention provides diagnostic systems for the controlled release of markers and therapeutic systems for the controlled release of therapeutic agents.

### Example 10

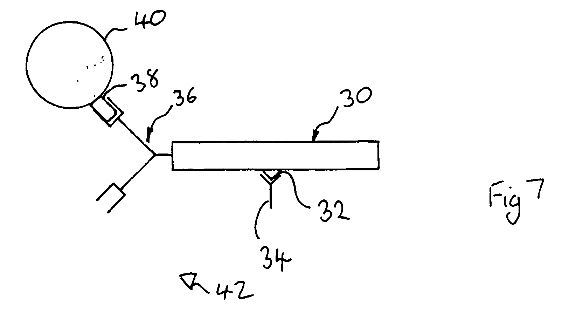

Fig. 7 shows another modified peptide 30 in accordance with the invention. The peptide comprises an active site 32 (e.g the conferring cytolysis ability) which is "cloaked" by binder 34 (e.g an antibody) as previously described. Conjugated to the peptide 30 is an antibody 36. The antibody is specific for an antigen 38 carried by a cell 40 (e.g a cancer cell). In use, the peptide is supplied to a patient with the first site 32 cloaked by the binder 34. The peptide 30 becomes bound to the cell 40 through antibody 36 binding to antigen 38. An activator 42 is then supplied to the patient which competes with the site 32 for binding to the binder 34 whereby the bound binder is released from the peptide. The active site 32 become functional. Where the action of the peptide is cytolyis it then causes cytolyis of the cells to which it is attached.

It will be appreciated that although the antibody 36 has been described as being conjugated to the peptide 30 the peptide could be engineered so that it is able to bind antigens. In other words antibody-like antigen binding function may be provided.

Various modifications of the invention in addition to those reported will be apparent to those skilled in the art and these also fall into the scope of the claims.

## Claims

1. A method of modifying the activity of a peptide, the method comprising:
a. taking a peptide comprising an active site and a non-active site, the non-active site being capable of conjugation with another particle or surface; and
b. substituting a first amino acid at a first site in the active site of the peptide with an amino acid which is modifiable by a reaction whereby the active site of the peptide is inactivated; and
c. substituting other chemically modifiable amino acids in the peptide with amino acids which are not modifiable by the reaction;
wherein the peptide is selected from or derived from lytic peptides, melittin, gramacidin, alamethicin, paradixin, ceropin, maganins, C18G, P14A, the peptide sequence:
LLQSLLSLLQSLLSLLQWLKRKRQQ, peptide sequence: GFFALIPKIISSPLFK, delta-Hemolysin, Mastopparan, Bombolitin, Crabolin, KLLKLLLKLLKLLLKLLLKLLKKLLKLLLKLLLKLLK, Defensins, antibacterial lytic peptides, Paradaxin, Cationic peptides, amphipathic peptides, GALA, KALA, HELP, LAGA, influenza Hemagglutinin peptide, HIV fusion peptide, peptide sequence: FEAALAEALAEALA, Signal sequences; and P-25
and wherein when the peptide is exposed to the reaction, the active site is reactivated by modification at the first site.

2. A method according to claim 1 in which the activity of the peptide is cytolytic activity or membrane activity.

3. A method according to claim 1 or claim 2 wherein the reactivation of the active site is delayed reactivation.

4. A method according to claim 3 wherein the reactivation is delayed for at least 14 hours.

5. A method according to any preceding claim wherein the particle or surface is a lipid membrane.

6. A method according to claim 5 wherein the peptide is integrated with the lipid membrane.

7. A method according to any preceding claim in which the non-active site capable of conjugation is introduced into the peptide by substitution of at least one amino acid at a non-active site of the peptide.

8. A method according to any preceding claim in which all the other amino acids are replaced in the peptide.

9. A method according to any preceding claim in which the peptide is obtained or derived from natural peptides or polypeptides.

10. A method according to any one of claims 1 to 8 in which the peptide has been produced synthetically.

11. A method according to any preceding claim in which the active site of the peptide is modified using an ionisable group, a redox sensitive group, an affinity ligand, an enzyme substrate, a light sensitive group, a pH sensitive group or a chemical group sensitive to physical, chemical or biochemical effects.

12. A method according to any preceding claim in which the peptide is modified by the replacement of amino acids with other amino acids or groups whereby binding of the peptide to a lipid membrane is improved.

## Patentansprüche

1. Ein Verfahren des Abwandelns der Aktivität eines Peptides, wobei das Verfahren aufweist:
a. Nehmen eines Peptides, das eine aktive Stelle und eine nicht-aktive Stelle aufweist, wobei die nicht-aktive Stelle zur Konjugation mit einem anderen Teilchen oder Oberfläche in der Lage ist; und
b. Ersetzen einer ersten Aminosäure an einer ersten Stelle in der aktiven Stelle des Peptides durch eine Aminosäure, die durch eine Reaktion abwandelbar ist, wodurch die aktive Stelle des Peptides inaktiviert wird; und
c. Ersetzen anderer chemisch abwandelbarer Aminosäuren in dem Peptid durch Aminosäuren, die nicht durch die Reaktion abwandelbar sind, wobei das Peptid ausgewählt oder abgeleitet ist aus lytischen Peptiden, Melittin, Gramacidin, Alamethicin, Paradixin, Ceropin, Maganinen, C18G, P14A, der Peptidfolge:
LLQSLLSLLQSLLSLLQWLKRKRQQ, Peptidfolge: GFFALIPKIISSPLFK, delta-Hämolysin, Mastopparan, Bombolitin, Crabolin, KLLKLLLKLLKLLLKLLLKLLKKLLKLLLKLLLKLLK, Defensinen, antibakteriellen lytischen Peptiden, Paradaxin, kationischen Peptiden, amphipatischen Peptiden, GALA, KALA, HELP, LAGA, Grippe-Hämagglutinin-Peptid, HIV-Verschmelzungspeptid, der Peptidfolge: FEAALAEALAEALA, Signalfolgen, und P-25 und worin, wenn das Peptid der Reaktion ausgesetzt wird, die aktive Stelle durch Abwandlung an der ersten Stelle reaktiviert wird.

2. Ein Verfahren nach Anspruch 1, in dem die Aktivität des Peptides eine zyklolytische Aktivität oder Membranaktivität ist.

3. Ein Verfahren nach Anspruch 1 oder 2, worin die Reaktivierung der aktiven Stelle verzögerte Reaktivierung ist.

4. Ein Verfahren nach Anspruch 3, worin die Reaktivierung um mindestens 14 Stunden verzögert ist.

5. Ein Verfahren nach irgendeinem vorhergehenden Anspruch, worin das Teilchen oder die Oberfläche eine Lipidmembran ist.

6. Ein Verfahren nach Anspruch 5, worin das Peptid mit der Lipidmembran integriert ist.

7. Ein Verfahren nach irgendeinem vorhergehenden Anspruch, in dem die nicht-aktive Stelle, die der Konjugation fähig ist, in das Peptid durch Ersetzen mindestens einer Aminosäure an einer nicht-aktiven Stelle des Peptides eingeführt wird.

8. Ein Verfahren nach irgendeinem vorhergehenden Anspruch, in dem all die anderen Aminosäuren ersetzt werden.

9. Ein Verfahren nach irgendeinem vorhergehenden Anspruch, in dem das Peptid aus natürlichen Peptiden oder Polypeptiden erhalten oder abgeleitet ist.

10. Ein Verfahren nach irgendeinem der Ansprüche 1 - 8, in dem das Peptid synthetisch erzeugt worden ist.

11. Ein Verfahren nach irgendeinem vorhergehenden Anspruch, in dem die aktive Stelle des Peptids durch Verwenden einer ionisierbaren Gruppe, einer redoxempfindlichen Gruppe, eines Affinitäts-Liganden, eines Enzymsubstrates, einer lichtempfindlichen Gruppe, einer pH-empfindlichen Gruppe oder einer chemischen Gruppe, die auf physikalische, chemische oder bio-chemische Effekte empfindlich ist, abgewandelt wird.

12. Ein Verfahren nach irgendeinem vorhergehenden Anspruch, in dem das Peptid durch die Ersetzung von Aminosäuren durch andere Aminosäuren oder Gruppen abgewandelt wird, wodurch die Bindung des Peptids an eine Lipidmembran verbessert wird.

## Revendications

1. Procédé de modification de l'activité d'un peptide, le procédé comprenant :
a. le prélèvement d'un peptide comprenant un site actif et un site non actif, le site non actif pouvant être conjugué à une autre particule ou surface; et
b. la substitution d'un premier acide aminé dans une première position dans le site actif du peptide par un acide aminé qui est modifiable via une réaction, de sorte que le site actif du peptide soit inactivé; et
c. la substitution d'autres acides aminés chimiquement modifiables dans le peptide par des acides aminés qui ne sont pas modifiables par la réaction; dans lequel le peptide est choisi ou dérivé du groupe constitué des peptides lytiques, de la mélittine, de la gramicidine, de l'alaméthicine, de la pardixine, de la cécropine, des magainines, du C18G, du P14A, de la séquence peptidique LLQSLLSLLQSLLSLLQWLKRKRQQ, de la séquence peptidique GFFALIPKIISSPLFK, de l'hémolysine delta, du mastoparan, de la bombolitine, de la carboline, de KLLKLLLKLLKLLLKLLLKLLKKLLKLLLKLLLKLLK, des défehsines, des peptides lytiques antibactériens, de la paradaxine, des peptides cationiques, des peptides amphipathiques, de GALA, de KALA, de HELP, de LAGA, du peptide de l'hémagglutinine du virus de la grippe, du peptide de fusion du VIH, de la séquence peptidique FEAALAEALAEALA, des séquences de signal et du P-25
et dans lequel, lorsque le peptide est exposé à la réaction, le site actif est réactivé par modification dans la première position.

2. Procédé selon la revendication 1, dans lequel l'activité du peptide est une activité cytolytique ou une activité membranaire.

3. Procédé selon la revendication 1 ou 2, dans lequel la réactivation du site actif est une réactivation retardée.

4. Procédé selon la revendication 3, dans lequel la réactivation est retardée pendant au moins 14 heures.

5. Procédé selon l'une quelconque des revendications précédentes, dans lequel la particule ou la surface est une membrane lipidique.

6. Procédé selon la revendication 5, dans lequel le peptide est intégré à la membrane lipidique.

7. Procédé selon l'une quelconque des revendications précédentes, dans lequel le site non actif capable de se conjuguer est introduit dans le peptide par substitution d'au moins un acide aminé dans un site non actif du peptide.

8. Procédé selon l'une quelconque des revendications précédentes, dans lequel tous les autres acides aminés sont remplacés dans le peptide.

9. Procédé selon l'une quelconque des revendications précédentes, dans lequel le peptide est obtenu ou dérivé de peptides ou de polypeptides naturels.

10. Procédé selon l'une quelconque des revendications 1 à 8, dans lequel le peptide a été produit de manière synthétique.

11. Procédé Selon l'une quelconque des revendications précédentes, dans lequel le site actif du peptide est modifié au moyen d'un groupement ionisable, d'un groupement à sensibilité redox, d'un ligand d'affinité, d'un substrat enzymatique, d'une groupement photosensible, d'un groupement sensible au pH ou d'un groupement chimique sensible aux effets physiques, chimiques ou biochimiques.

12. Procédé selon l'une quelconque des revendications précédentes, dans lequel le peptide est modifié par le remplacement d'acides aminés par d'autres acides aminés ou groupements, de sorte que la liaison du peptide à une membrane lipidique est améliorée.
